(19) Europäisches Patentamt
European Patent Office
Office européen des brevets

(11) EP 4 118 960 A1

(12) EUROPEAN PATENT APPLICATION
published in accordance with Art. 153(4) EPC

(43) Date of publication:
**18.01.2023 Bulletin 2023/03**

(21) Application number: **21767132.0**

(22) Date of filing: **03.03.2021**

(51) International Patent Classification (IPC):
***A01K 29/00*** (2006.01)

(52) Cooperative Patent Classification (CPC):
**A01K 29/00**

(86) International application number:
**PCT/JP2021/008052**

(87) International publication number:
**WO 2021/182204 (16.09.2021 Gazette 2021/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.03.2020 JP 2020040117**

(71) Applicant: **Nitto Denko Corporation**
**Ibaraki-shi, Osaka 567-8680 (JP)**

(72) Inventors:
- **KOZONOI, Nobuyuki**
**Ibaraki-shi, Osaka 567-8680 (JP)**
- **KIGAWA, Yoichi**
**Ibaraki-shi, Osaka 567-8680 (JP)**
- **LI, Wenjing**
**Ibaraki-shi, Osaka 567-8680 (JP)**
- **KASEDA, Yugo**
**Ibaraki-shi, Osaka 567-8680 (JP)**
- **YOKOYAMA, Hiromi**
**Ibaraki-shi, Osaka 567-8680 (JP)**
- **HIRAMOTO, Ryosuke**
**Ibaraki-shi, Osaka 567-8680 (JP)**

(74) Representative: **Müller-Boré & Partner Patentanwälte PartG mbB**
**Friedenheimer Brücke 21**
**80639 München (DE)**

## (54) ANOMALY SENSING SYSTEM

(57) An abnormality detecting system includes a first identification unit configured to identify a state of an animal that is a monitoring target in each time range, based on time-series data from a motion sensor placed on a predetermined portion of the animal that is a monitoring target; a first calculation unit configured to calculate a transition probability from the state at a predetermined timing of each time range identified by the first identification unit to a next state; and a determining unit configured to determine that an abnormality of the animal that is a monitoring target is detected when a score calculated based on the transition probability to the next state satisfies a predetermined condition.

ACCELERATION
510
Y
Z
X
TIME
1 [SEC]
1 [SEC]
1 [SEC]
520
530
STATE I
STATE V
STATE IV
541
542
540
NEXT STATE
STATE I
STATE II
STATE III
STATE DCCXXIX
TOTAL
PRESENT STATE
TRANSITION PROBABILITY = EACH TRANSITION FREQUENCY / TOTAL NUMBER OF TRANSITION FREQUENCY
STATE TRANSITION PROBABILITY DATA OF HEALTHY CATTLE
FIG.5



Processed by Luminess, 75001 PARIS (FR)

## Description

[Technical Field]

The present invention relates to an abnormality detecting system.

[Background Art]

Generally speaking, a workflow from breeding through marketing of a cow is broadly divided into multiple steps (for example, grass-feeding, fattening, meat-processing, and so forth). Of these, the fattening process is carried out in a certain area called a feedlot, and therefore, a cow is susceptible to a bovine respiratory disease (BRD) or a bovine respiratory disease complex (BRDC).

In this regard, for example, Non-Patent Document 1 below proposes a system for automatically detecting a cow that has actually developed a BRD (or BRDC) among cows infected with the BRD (or BRDC). The system allows earliest identification of the cow that has actually developed the BRD (or BRDC).

[Prior art documents]

[Non-patent documents]

[Non-Patent Document 1] Cargill, "Cargill brings facial recognition capability to farmers through strategic equity investment in Cainthus", [online], January 31, 2018 [retrieved February 28, 2020], Internet [URL: https://www.cargill.com/2018/cargill-brings-facial-recognition-capability-to-farmers]

[Summary of the Invention]

[Problem to be Solved by the Invention]

In this regard, detecting an early warning of (or some abnormality concerning) a BRD (or BRDC) prior to an onset of the BRD (or BRDC) in a cow that is a monitoring target may reduce various costs required due to the onset of the BRD (or BRDC) or an increase in severity of the BRD (or BRDC).

In one aspect, an object is to provide an abnormality detecting system for detecting an abnormality in an animal that is a monitoring target.

[Means for Solving the Problem]

According to an aspect of the present disclosure, an abnormality detecting system includes

a first identification unit configured to identify a state of an animal that is a monitoring target in each time range, based on time-series data from a motion sensor placed on a predetermined portion of the animal that is a monitoring target;

a first calculation unit configured to calculate a transition probability from the state at a predetermined timing of each time range identified by the first identification unit to a next state; and

a determining unit configured to determine that an abnormality of the animal that is a monitoring target is detected when a score calculated based on the transition probability to the next state satisfies a predetermined condition.

[Advantageous Effects of the Invention]

An abnormality detecting system for detecting an abnormality in an animal that is a monitoring target can be provided.

[Brief Description of Drawings]

Fig. 1 is a first diagram illustrating an example of a system configuration of an abnormality detecting system and a functional configuration of a server apparatus.
Fig. 2 is a diagram illustrating an example of a hardware configuration of the server apparatus.
Fig. 3 is a diagram illustrating a specific example of a process of a data obtaining unit.
Fig. 4 is a diagram illustrating details of a functional configuration of a reference data calculation unit.
Fig. 5 is a diagram illustrating a specific example of process of the reference data calculation unit.
Fig. 6 is a first diagram illustrating details of a functional configuration of an analysis unit.
Fig. 7 is a first diagram illustrating a specific example of a process of the analysis unit.
Fig. 8 is a diagram illustrating a specific example of a process of an early warning detecting unit.
Fig. 9 is a flowchart illustrating a flow of a reference data calculation process.
Fig. 10 is a first flowchart illustrating a flow of an analysis process.
Fig. 11 is a flowchart illustrating a flow of an early warning detecting process.
Fig. 12 is a second diagram depicting an example of the system configuration of the abnormality detecting system and the functional configuration of the server apparatus.
Fig. 13 is a second diagram illustrating details of the functional configuration of the analysis unit.
Fig. 14 is a second diagram illustrating a specific example of the process of the analysis unit.
Fig. 15 is a second flowchart illustrating the flow of the analysis process.

[Mode for Carrying Out the Invention]

Hereinafter, each embodiment will be described with reference to the accompanying drawings. In the present specification and the drawings, for the components having substantially the same functional configurations, the duplicate descriptions are omitted by providing the same reference numerals thereto.

[First embodiment]

<System configuration of abnormality detecting system and server apparatus>

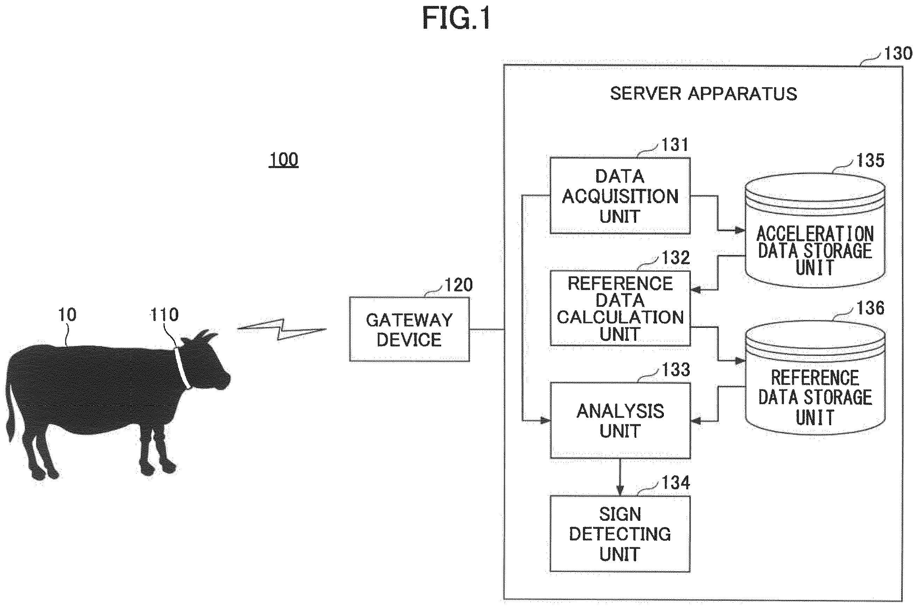

First, the system configuration of the abnormality detecting system and the functional configuration of the server apparatus will be described. Fig. 1 is a first diagram illustrating an example of the system configuration of the abnormality detecting system and the functional configuration of the server apparatus.

The abnormality detecting system 100 is a system for detecting an early warning of (or some abnormality concerning) a BRD (or BRDC) in any cow in a feedlot before the cow develops the BRD (or BRDC) during a fattening process.

As depicted in Fig. 1, the abnormality detecting system 100 includes a measuring device 110, a gateway device 120, and the server apparatus 130. In the abnormality detecting system 100, the measuring device 110 and the gateway device 120 are interconnected via wireless communication, and the gateway device 120 and the server apparatus 130 are interconnected via a network (not depicted).

The measuring device 110 is a motion sensor (in the present embodiment, an acceleration sensor) of three dimensions (an X-axis direction, a Y-axis direction, and a Z-axis direction) attached to a predetermined portion of a cow 10 (in the example of Fig. 1, the neck of the cow 10). The X-axis direction is, for example, a direction along a body surface of the neck of the cow 10, which is a direction along a circumference of the neck, and the Y-axis direction is, for example, a direction along the body surface of the neck of the cow 10, which is a direction to the body relative to the head. The Z-axis direction is, for example, a direction perpendicular to the body surface of the neck of the cow 10.

The measuring device 110 measures time-series three-dimensional acceleration data at a predetermined sampling frequency and transmits the data to the gateway device 120.

The gateway device 120 transmits the time-series three-dimensional acceleration data transmitted from the measuring device 110 to the server apparatus 130.

The server apparatus 130 detects an early warning of (or some abnormality concerning) a BRD (or BRDC) in any cow in a feedlot before the cow develops the BRD (or BRDC). An abnormality detecting program is installed in the server apparatus 130, and when the program is executed, the server apparatus 130 functions as a data obtaining unit 131, a reference data calculation unit 132, an analysis unit 133, and an early warning detecting unit 134.

The data obtaining unit 131 stores time-series data of healthy cow among the time-series data representing the three-dimensional acceleration transmitted from the gateway device 120 in the acceleration data storing unit 135.

The reference data calculation unit 132 reads out the time-series data representing the three-dimensional acceleration of healthy cow stored in the acceleration data storing unit 135 and calculates state transition probability data of healthy cow (will be described in detail below). The reference data calculation unit 132 stores the state transition probability data of healthy cow in the reference data storing unit 136 as reference data.

The analysis unit 133 calculates state transition probability data of the cow that is a monitoring target based on time-series data of the cow that is a monitoring target among the time-series data representing the three-dimensional acceleration acquired by the data obtaining unit 131. The analysis unit 133 calculates a score indicating how far the state transition probability data of the cow that is a monitoring target deviated from the state transition probability data of healthy cow (that is, an abnormality of the state transition).

The early warning detecting unit 134 (determining unit) acquires the score calculated by the analysis unit 133 as data indicating the abnormality of the cow that is a monitoring target and determines whether an early warning (or some abnormality) is detected before development of BRD (or BRDC) in the cow that is a monitoring target. When it is determined that an early warning (or some abnormality) has been detected, the early warning detecting unit 134 notifies a user.

In addition, for example, when data indicating abnormality of the cow that is a monitoring target exceeds a predetermined threshold value for a plurality of consecutive days (for example, two days), the early warning detecting unit 134 determines that an early warning (or some abnormality) has been detected.

<Hardware configuration of server apparatus>

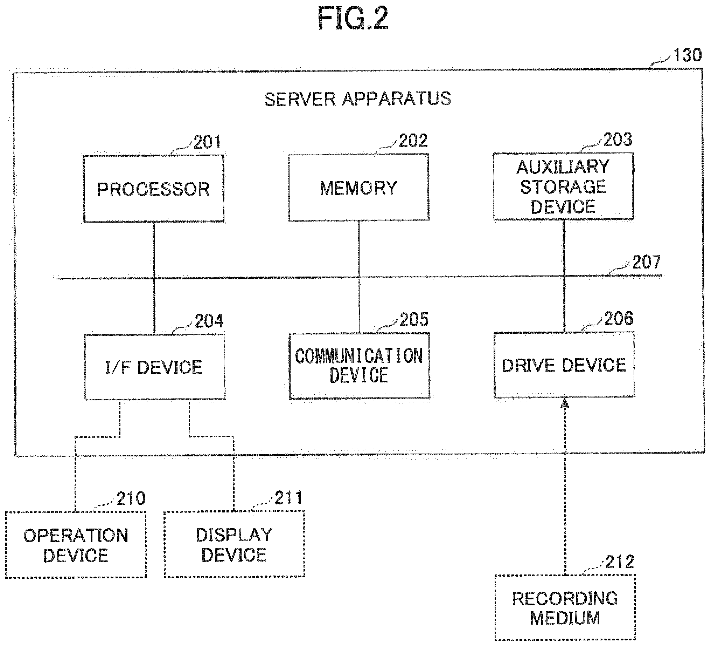

Next, a hardware configuration of the server apparatus 130 will be described. Fig. 2 is a diagram illustrating an example of a hardware configuration of the server apparatus. As depicted in Fig. 2, the server apparatus 130 includes a processor 201, a memory 202, an auxiliary storage device 203, an interface (I/F) device 204, a communication device 205, and a drive device 206. These hardware elements of the server apparatus 130 are interconnected via a bus 207.

The processor 201 includes various arithmetic and logic operation units such as a central processing unit (CPU), a graphics processing unit (GPU), and so forth. The processor 201 reads various programs (for example, an abnormality detecting program, and so forth), writes the programs into the memory 202, and executes the programs.

The memory 202 includes main storage devices such as a read-only memory (ROM), a random access memory (RAM), and the like. The processor 201 and the memory 202 form what is known as a computer, and when the processor 201 executes the various programs read and written onto the memory 202, the computer implements the above-described functions.

The auxiliary storage device 203 stores the various programs and various data used when the various programs are executed by the processor 201. For example, the acceleration data storing unit 135 and the reference data storing unit 136 are implemented in the auxiliary storage device 203.

The I/F device 204 is a connection device that connects a manually-operating device 210, a display device 211, and the server apparatus 130, which are examples of an external apparatus/device. The I/F device 204 receives a manual operation performed on the server apparatus 130 through the manually-operating device 210. The I/F device 204 outputs the result of a process performed by the server apparatus 130 and displays the result through the display device 211.

The communication device 205 is a communication device for communicating with other apparatuses/devices. In the server apparatus 130, the communication device 205 is used to communicate with the gateway device 120 that is another device.

The drive device 206 is a device for setting a recording medium 212. The recording medium 212 is a medium for optically, electrically, or magnetically recording information, such as a CD-ROM, a flexible disk, a magneto-optical disk, or the like. The recording medium 212 may be a semiconductor memory or the like that electrically records information, such as a ROM, a flash memory, or the like.

The various programs installed in the auxiliary storage device 203 are installed, for example, as a result of a distributed recording medium 212 being set in the drive device 206 and the various programs recorded in the recording medium 212 being read out by the drive device 206. Alternatively, the various programs installed in the auxiliary storage device 203 may have been installed by downloading the various programs from a network via the communication device 205.

<Example of process by data obtaining unit>

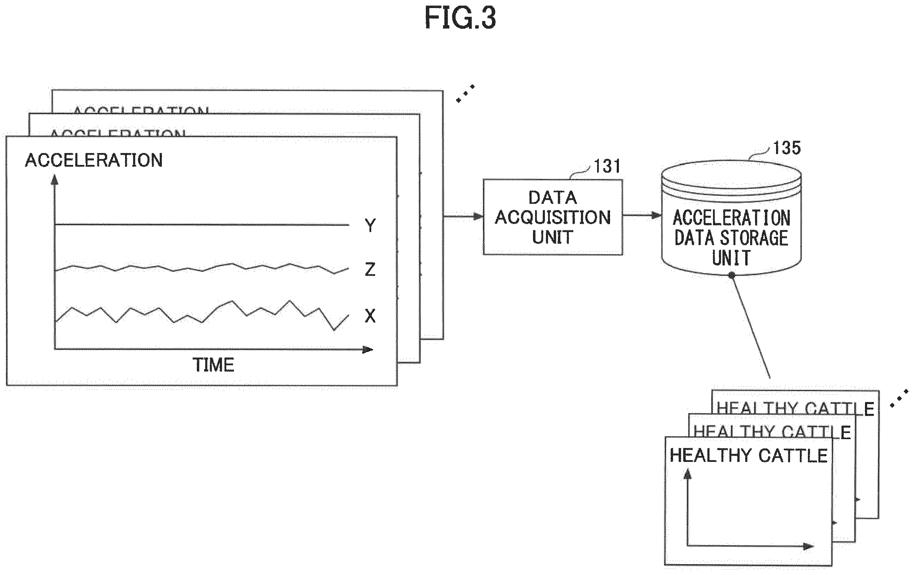

Next, a specific example of the process of the data obtaining unit 131 among the process of the units executed by the server apparatus 130 will be described. Fig. 3 is a diagram illustrating a specific example of process of the data obtaining unit. As shown in Fig. 3, the data obtaining unit 131 acquires a set of time-series data (time-series X-axis-directional acceleration data, time-series Y-axis-directional acceleration data, and time-series Z-axis-directional acceleration data) for various cows transmitted from the gateway device 120.

The data obtaining unit 131 stores a set of time-series data for healthy cow in the acceleration data storing unit 135.

<Details of Functional Configuration of Reference data Calculation Unit>

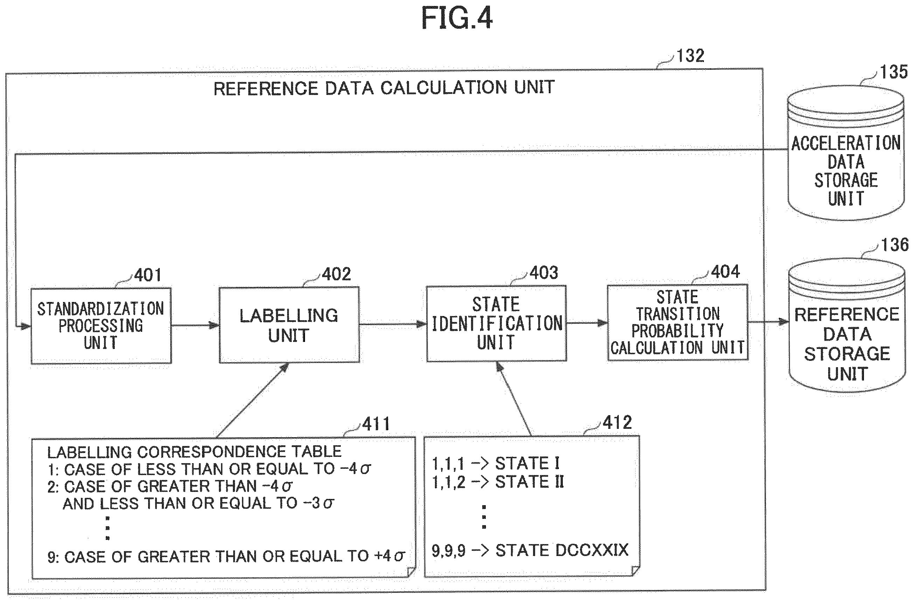

Next, a functional configuration of the reference data calculation unit 132 among the functional configurations of the units implemented by the server apparatus 130 will be described in detail. Fig. 4 is a diagram illustrating a detailed functional configuration of the reference data calculation unit. As shown in Fig. 4, the reference data calculation unit 132 includes a standardization processing unit 401 (the second standardization processing unit), a labeling unit 402 (the second labeling unit), a state identification unit 403 (the second identification unit), and a state transition probability calculation unit 404 (the second calculation unit).

The standardization processing unit 401 reads out the set of time-series data for healthy cow from the acceleration data storing unit 135, performs the standardization process for the time-series data in each axis, and notifies the labeling unit 402 of the standardized data.

The standardization process refers to a process of dividing the time-series data of each axis by a predetermined time range (for example, 1 [SEC]) and calculating a variation of the time-series data of each axis in each time range. That is, the standardized data is composed of calculation results of the variation of the time-series data of each axis in each time range.

The labeling unit 402 performs a labeling process on the standardized data. In the labeling unit 402, the labeling process is performed, i.e.,

> when the calculation result of the variation of the time-series data of each axis in each time range included in the standardized data is $-4\sigma$ or less, "1" is assigned to the standardized data;
> when the variation is greater than $-4\sigma$ and less than or equal to $-3\sigma$, "2" is assigned;
> when the variation is greater than $-3\sigma$ and less than or equal to $-2\sigma$, "3" is assigned;
> when the variation is greater than $-2\sigma$ and less than or equal to $-1\sigma$, "4" is assigned;
> when the variation is greater than $-1\sigma$ and less than $+1\sigma$, "5" is assigned;
> when the variation is greater than or equal to $+1\sigma$ and less than $+2\sigma$, "6" is assigned;
> when the variation is greater than or equal to $+2\sigma$ and less than $+3\sigma$, "7" is assigned;
> when the variation is greater than or equal to $+3\sigma$ and less than $+4\sigma$, "8" is assigned; and
> when the variation is greater than or equal to $+4\sigma$, "9" is assigned (see reference numeral 411) .

The state identification unit 403 identifies a state of healthy cow in each time range based on the labeling data generated by performing the labeling process on the time-series data of each axis in each time range. For example, in each time range, when the result of the labeling process for the time-series data in the X-axis direction ($L_x$), the result of the labeling process for the time-series data in the Y-axis direction ($L_Y$), and the result of the labeling process for the time-series data in the Z-axis direction ($L_Z$),

are ($L_X$=1, $L_Y$=1, $L_Z$=1) respectively, the state of the cow is identified as state I;
are ($L_X$=1, $L_Y$=1, $L_Z$=2) respectively, the state of the cow is identified as state II;
• • • are ($L_X$=9, $L_Y$=9, $L_Z$=9) respectively, the state of the cow is identified as state DCCXXIX (see reference numeral 412).

The state transition probability calculation unit 404 calculates a transition probability indicating to which state a state of healthy cow at a predetermined timing of each time range is changed in the next time range. For example, when the state of the cow 10 in the current time range is "state I", the state of the cow 10, in the next time range, transitions to one of 729 states of "state I" to "state DCCXXIX". The state transition probability calculation unit 404 counts states transitioned from "state I" in the next time range for each state of the transition destination for a certain period of time. The state transition probability calculation unit 404 divides a number of transitions to each state by the total number of transitions to any state. Accordingly, the state transition probability calculation unit 404 calculates the transition probability from the current state to the next state, for example, for the cow 10.

The state transition probability calculation unit 404 stores the state transition probability data generated by calculating the transition probabilities for, for example, all states of the cow 10 as the transition source in the reference data storing unit 136 as reference data.

As is obvious from the above description, it can be said that the state transition probability data generated by the state transition probability calculation unit 404 is data indicating a tendency of movement of healthy cow.

<Specific Example of Process by Reference Data Calculation Unit>

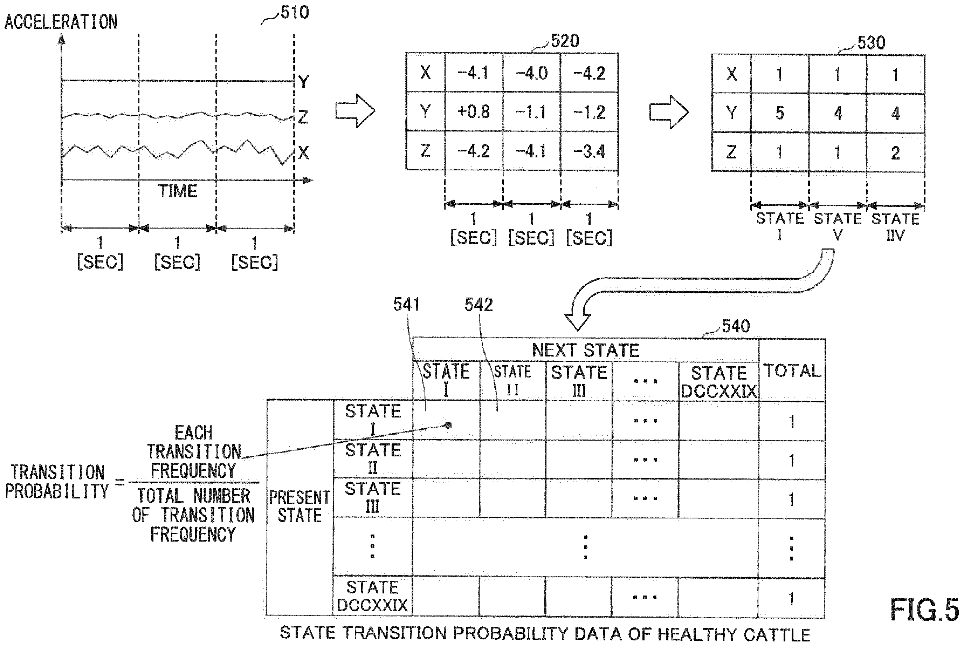

Next, a specific example of the process of the reference data calculation unit 132 among the process of the units executed by the server apparatus 130 will be described. Fig. 5 is a diagram illustrating a specific example of process of the reference data calculation unit.

As shown in Fig. 5, when a set of time-series data 510 for healthy cow is acquired, the standardization processing unit 401 performs the standardization process to generate the standardized data 520. The example of Fig. 5 shows that spacing between dashed lines of the set of time-series data 510 is a time range of 1 [SEC]. The value included in the standardized data 520 indicates the variation of the time-series data calculated for each of the X-axis, Y-axis, and Z-axis for every 1 [SEC].

As shown in Fig. 5, the labeling process is performed for the standardized data 520 by the labeling unit 402, and the labeling data 530 is generated. The state identification unit 403 identifies the state in each time range of healthy cow based on the result of the labeling process of each axis in each time range constituting the labeling data 530 (states I, V, and IV).

In the example shown in Fig. 5, the labeling process is performed using 9 kinds of labels from 1 to 9, so that the combination of the results of the labeling process for the X-axis, Y-axis, and Z-axis is 729 (=9x9x9). That is, any state from state I to state DCCXXIX is identified as a state in each time range.

Also, as shown in Fig. 5, the state transition probability calculation unit 404 calculates the transition probability from a state at the predetermined timing of each time range to the next state for healthy cow based on the data (data for a certain period of time) indicating a state in each time range of healthy cow. Thus, the state transition probability calculation unit 404 generates state transition probability data 540 of healthy cow. The state transition probability data 540 of Fig. 5 is generated by arranging the current state (729 states) of healthy cow vertically, and the next state (729 states) of healthy cow horizontally, and storing the transition probabilities to the next state in each column.

For example, in column 541, for the data for a certain period of time indicating states in each time range of healthy cow, the transition probability from "state I" to "state I" calculated by:

counting a number of transitions (individual number of transitions) in which the current state in the time range is "state I" and the state in the next time range remains "state I";
counting the total number of transitions, in which the current state in the time range is "state I", and the state in the next time range is transitioned to any state from "state I" to "state DCCXXIX"; and
dividing individual transition probabilities by the total number of transitions, is stored.

Similarly, in column 542, for the data for a certain period of time indicating states in each time range of healthy cow, the transition probability from "state I" to "state II" calculated by:

counting a number of transitions (individual number of transitions) in which the current state in the time range is "state I" and the state in the next time range is transitioned to "state II";
counting the total number of transitions, in which the current state in the time range is "state I", and the state in the next time range is transitioned to any state from "state I" to "state DCCXXIX"; and
dividing individual transition probabilities by the total number of transitions, is stored.

Hereinafter, the same process is performed for the transition destination states of from "state III" to "state DCCXXIX", and for the transition source states of from "state II" to "state DCCXXIX", so that the state transition probability data 540 of healthy cow is generated. Incidentally, as shown in Fig. 5, the sum of the transition probabilities with the same transition source and different transition destinations is "1" (see item of information included in the state transition probability data 540 = "Total").

The state transition probability data 540 generated for healthy cow is stored in the reference data storing unit 136 as reference data.

<Details of Functional Configuration of Analysis Unit>

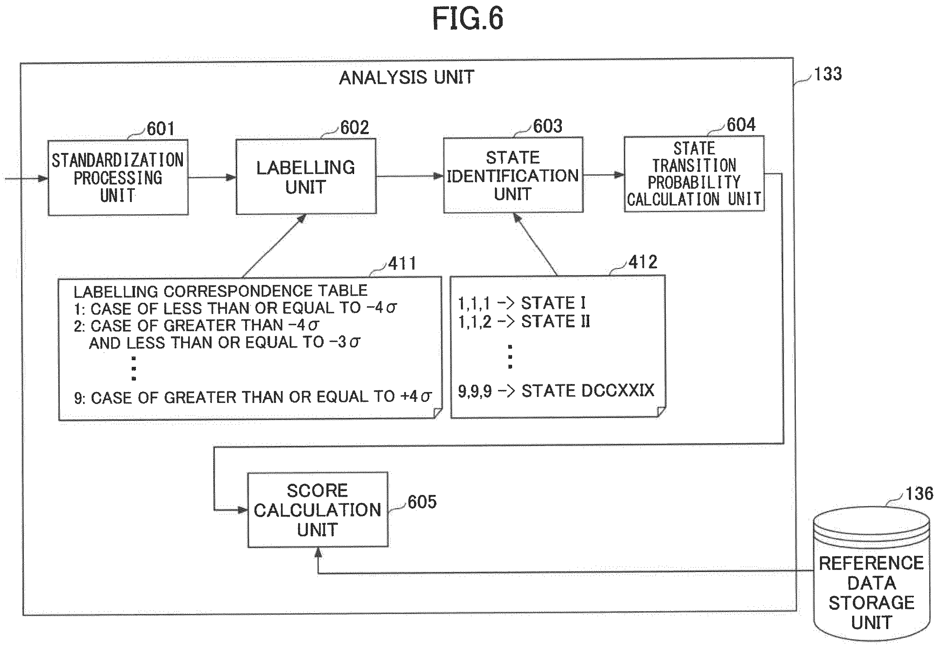

Next, the functional configuration of the analysis unit 133 among the functional configurations of units implemented by the server apparatus 130 will be described in detail. Fig. 6 is a diagram illustrating a detailed functional configuration of the analysis unit. As shown in Fig. 6, the analysis unit 133 includes a standardization processing unit 601 (first standardization processing unit) and a labeling unit 602 (first labeling unit). The analysis unit 133 includes a state identification unit 603 (first identification unit), a state transition probability calculation unit 604 (first calculation unit), and a score calculation unit 605 (first score calculation unit).

Functions of the respective units of the standardization processing unit 601 to the state transition probability calculation unit 604 are the same as those of the respective units of the standardization processing unit 401 to the state transition probability calculation unit 404 of the reference data calculation unit 132 described with reference to Fig. 4. Accordingly, the score calculation unit 605 will be described here.

The score calculation unit 605 acquires the state transition probability data of the cow that is a monitoring target generated by the state transition probability calculation unit 604. The score calculation unit 605 reads out the state transition probability data of healthy cow from the reference data storing unit 136. The score calculation unit 605 calculates, based on the state transition probability data of the cow that is a monitoring target and the state transition probability data of healthy cow, a score using the following equation (1),

(Equation 1)

$$\text{score} = -\log(P \circ X).$$

In equation (1) above, P represents the state transition probability data of healthy cow, and X represents the state transition probability data of the cow that is a monitoring target. Furthermore, o indicates an Amadal product. Based on the above equation (1), the score calculation unit 605 calculates for each transition probability a score indicating how far the state transition probability data of the cow that is a monitoring target deviates from the state transition probability data of healthy cow (that is, an abnormality of the state transition).

In addition, the score calculation unit 605 extracts the maximum score from among all of the scores calculated for each of the transition probabilities, and outputs it as data indicating the abnormality of the cow that is a monitoring target at the relevant date.

As is obvious from the above description, the state probability data generated by the state transition probability calculation unit 604 can be said to be data indicating a tendency of movement of the cow that is a monitoring target. In this embodiment, under the assumption that a change occurs in the tendency of movement of the cow that is a monitoring target before the development of BRD (or BRDC), the above score is calculated to detect the change, in contrast to the tendency of movement of healthy cow.

<Specific Example of Process in Analysis Unit>

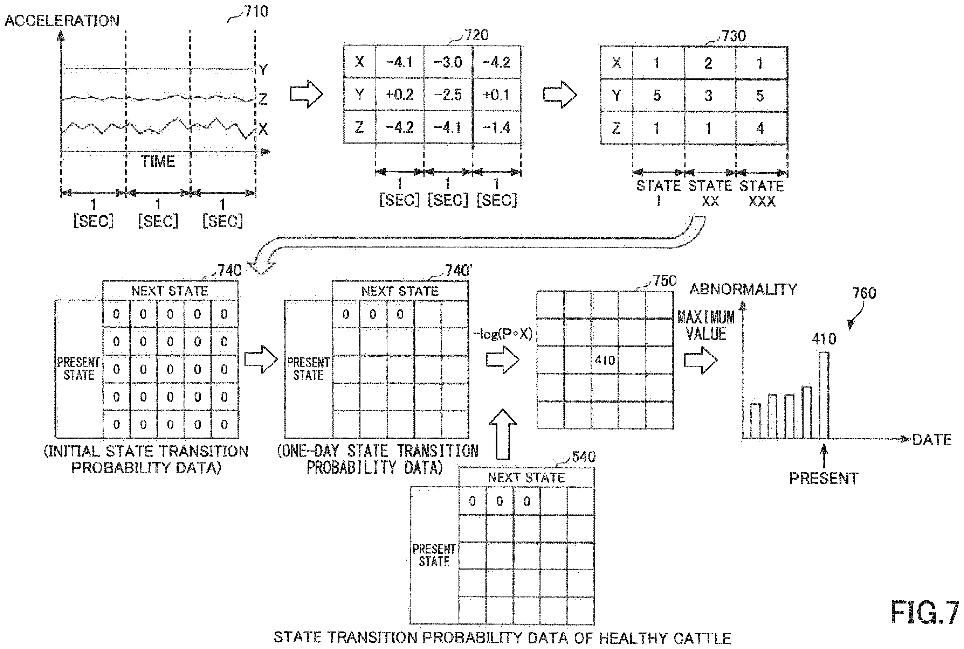

Next, a specific example of the process of the analysis unit 133 among the process of the units executed by the server apparatus 130 will be described. Fig. 7 is a diagram illustrating a specific example of process of the analysis unit.

As shown in Fig. 7,

a set of time-series data 710,
standardized data 720, and
labeling data 730,

which are acquired or generated for the cow that is a monitoring target by the standardization processing unit 601, the labeling unit 602, the state identification unit 603, and the state transition probability calculation unit 604, are the same as

the set of time-series data 510,
the standardized data 520, and
the labeling data 530,

which are acquired or generated for healthy cow by the standardization processing unit 401, the labeling unit 402, the state identification unit 403, and the state transition probability calculation unit 404. Thus, the detailed description will be omitted here.

As shown in Fig. 7, the state transition probability calculation unit 604 generates the one-day state transition probability data 740' of the cow that is a monitoring target by accumulating data of one day for initial state transition probability data 740 of the cow that is a monitoring target. The initial state transition probability data 740 refers to state transition probability data in which the transition probability of each state is "0". In the example of Fig. 7, only five current states and five subsequent states are shown as the state transition probability data 740 and 740' due to the limited space in the figure.

The score calculation unit 605 reads out the state transition probability data 540 for healthy cow and uses the above equation (1) to calculate the score for each transition probability, as shown in Fig. 7, by generating the one-day state transition probability data 740' of the cow that is a monitoring target. Thus, score data 750 is generated.

As shown in Fig. 7, the score calculation unit 605 extracts the maximum score from among the scores for each of the transition probabilities included in the score data 750, and outputs the extracted maximum score as data indicating the abnormality of the cow that is a monitoring target at the relevant date. In Fig. 7, a graph 760 is a graphical representation of the data representing the abnormality at each date of the cow that is a monitoring target, with the horizontal axis representing the date and the vertical axis representing the abnormality. The graph 760 shows that the data indicating the current abnormality is "410".

<Specific Example of Process of Early warning detecting unit>

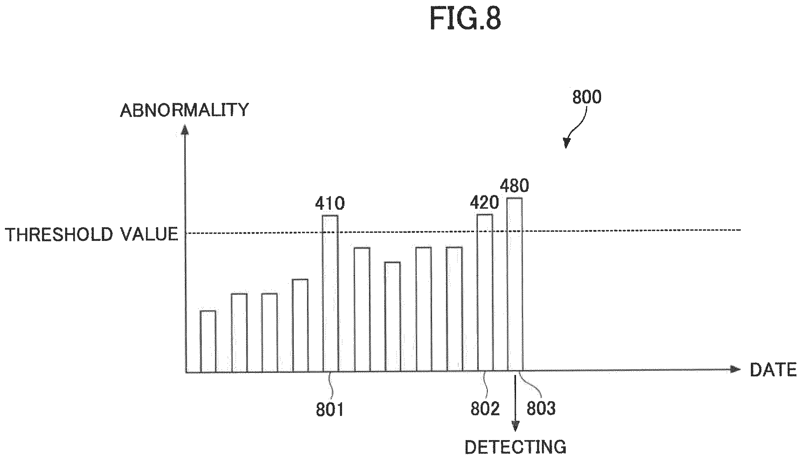

Next, a specific example of the process of the early warning detecting unit 134 among the process of the units executed by the server apparatus 130 will be described. Fig. 8 is a diagram illustrating a specific example of process of the early warning detecting unit. In Fig. 8, a graph 800, in the same manner as the graph 760, has a horizontal axis indicating date and a vertical axis indicating abnormality. The graph 800 also shows that the data representing the abnormality is greater than or equal to the threshold value at the date represented by the reference numeral 801, the reference numeral 802, and the reference numeral 803.

In addition, the graph 800 shows that, in the case of the date indicated by the reference numeral 801, since the data indicating the abnormality is greater than or equal to the threshold only in one day and is not consecutive, the early warning detecting unit 134 does not determine that an early warning (or some abnormality) has been detected.

On the other hand, the graph 800 shows that, in the case of the date indicated by the reference numeral 803, since the data indicating the abnormality is greater than or equal to the threshold value for consecutive two days, the early warning detecting unit 134 determines that an early warning (or some abnormality) has been detected.

<Flow of Reference data Calculation Process>

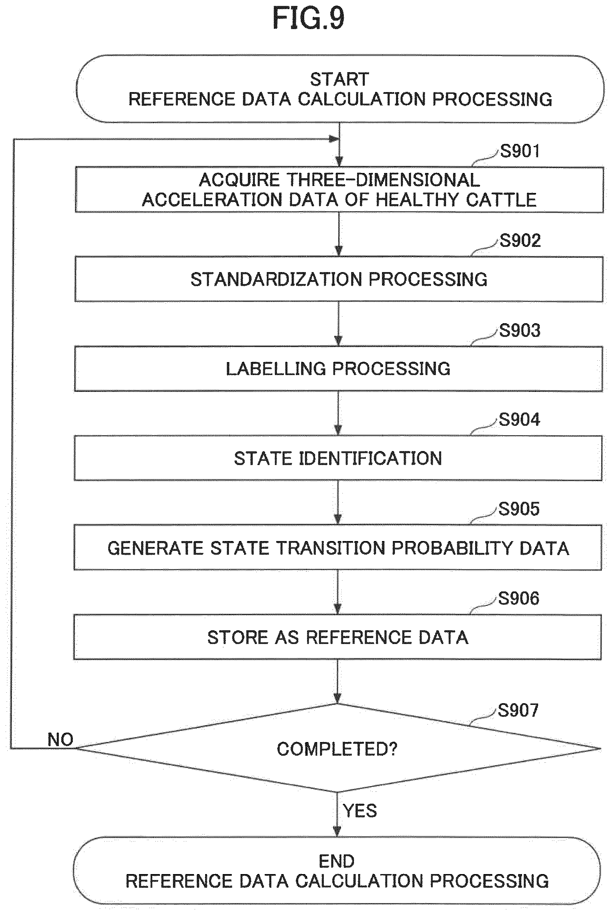

Next, a flow of the reference data calculation process performed by the server apparatus 130 will be described. Fig. 9 is a flowchart illustrating the flow of the reference data calculation process.

In step S901, the data obtaining unit 131 acquires time-series data indicating the three-dimensional acceleration of healthy cow.

In step S902, the standardization processing unit 401 of the reference data calculation unit 132 performs the standardization process for the time-series data representing the three-dimensional acceleration of healthy cow and generates the standardized data.

In step S903, the labeling unit 402 of the reference data calculation unit 132 performs the labeling process for the standardized data, and generates labeling data.

In step S904, the state identification unit 403 of the reference data calculation unit 132 identifies the state of each time range of healthy cow based on the labeling data.

In step S905, the state transition probability calculation unit 404 of the reference data calculation unit 132 generates state transition probability data of healthy cow based on data indicating the state of each time range of healthy cow.

In step S906, the state transition probability calculation unit 404 of the reference data calculation unit 132 stores the generated state transition probability data of healthy cow in the reference data storing unit 136 as the reference data.

In step S907, the state transition probability calculation unit 404 of the reference data calculation unit 132 determines whether the state transition probability data for a certain period of time of healthy cow has been generated. When it is determined in step S907 that the data is not generated (in the case of NO in step S907), the process returns to step S901.

On the other hand, when it is determined in step S907 that the data is generated (in the case of YES in step S907), the reference data calculation process ends.

<Flow of Analysis Process>

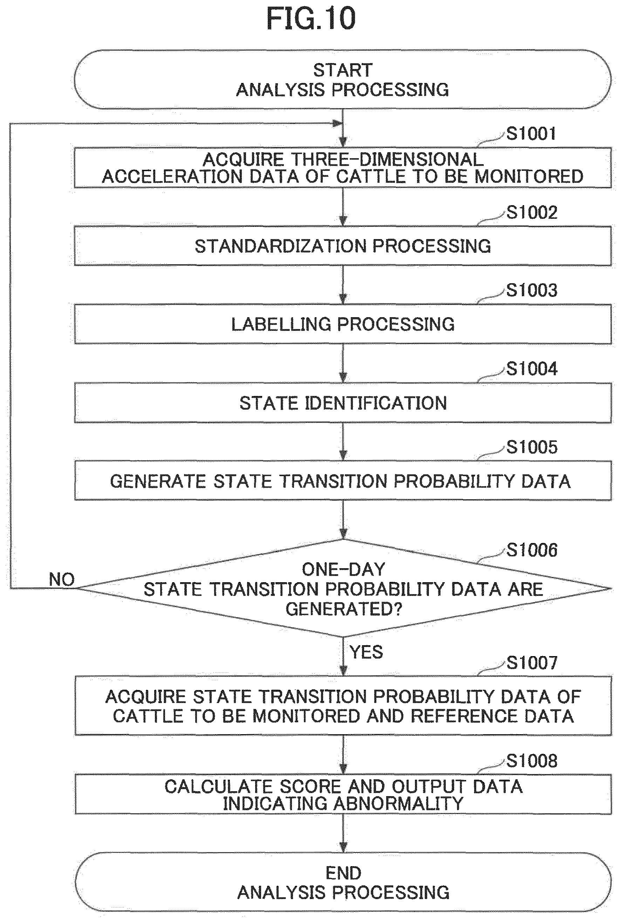

Next, the flow of the analysis process by the server apparatus 130 will be described. Fig. 10 is a first flowchart showing the flow of the analysis process and shows the one-day analysis process.

In step S1001, the data obtaining unit 131 acquires time-series data representing the three-dimensional acceleration of the cow that is a monitoring target.

In step S1002, the standardization processing unit 601 of the analysis unit 133 performs the standardization process for the time-series data representing the three-dimensional acceleration of the cow that is a monitoring target, and generates the standardized data.

In step S1003, the labeling unit 602 of the analysis unit 133 performs the labeling process for the standardized data and generates the labeling data.

In step S1004, the state identification unit 603 of the analysis unit 133 identify the state of each time range of healthy cow based on the labeling data.

In step S1005, the state transition probability calculation unit 604 of the analysis unit 133 generates the state transition probability data of the cow that is a monitoring target based on data indicating the state of the cow that is a monitoring target in each time range.

In step S1006, the state transition probability calculation unit 604 of the analysis unit 133 determines whether one-day state transition probability data of the cow that is a monitoring target has been generated. When it is determined in step S1006 that the one-day state transition probability data has not been generated (in the case of NO in step S1006), the process returns to step S1001.

On the other hand, when it is determined in step S1006 that the one-day state transition probability data has been generated (in the case of YES in step S1006), the process proceeds to step S1007.

In step S1007, the score calculation unit 605 of the analysis unit 133 acquires the one-day state transition probability data of the cow that is a monitoring target, and reads out the state transition probability data of healthy cow as reference data from the reference data storing unit 136.

In Step S1008, the score calculation unit 605 of the analysis unit 133 calculates a score for each transition probability based on the one-day state transition probability data of the cow that is a monitoring target and the state transition probability data of healthy cow read as reference data. Further, the score calculation unit 605 of the analysis unit 133 extracts the maximum score from among the scores calculated for each of the transition probabilities, and outputs it as data indicating the abnormality of the cow that is a monitoring target at the relevant date.

<Flow of Early warning detecting process>

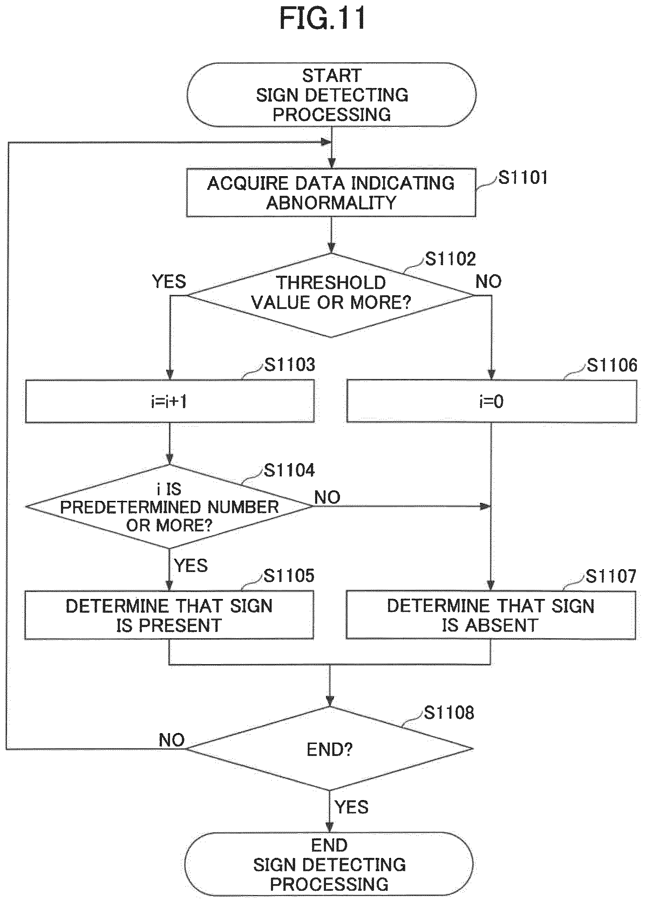

Next, the flow of the early warning detecting process by the server apparatus 130 will be described. Fig. 11 is a flowchart illustrating the flow of the early warning detecting process.

In step S1101, the early warning detecting unit 134 acquires data representing the abnormality of the cow that is a monitoring target, which is output every day from the score calculation unit 605 of the analysis unit 133.

In step S1102, the early warning detecting unit 134 determines whether the acquired data representing abnormality is greater than or equal to a predetermined threshold value. If it is determined in step S1102 that the acquired data representing abnormality is greater than or equal to the predetermined threshold value (in the case of YES in step S1102), the process proceeds to step S1103.

In step S1103, the early warning detecting unit 134 increments a counter i which counts the number of consecutive days. It is assumed that "0" is input as the initial value in counter i.

In step S1104, the early warning detecting unit 134 determines whether the counter i is greater than or equal to a predetermined number (for example, two days or more). When it is determined in step S1104 that the counter i is greater than or equal to the predetermined number (in the case of YES in step S1104), the process proceeds to step S1105.

In step S1105, the early warning detecting unit 134 determines that an early warning (or some abnormality) has been detected, and notifies the user thereof.

On the other hand, when it is determined in step S1104 that the counter i is less than the predetermined number (in the case of NO in step S1104), the process proceeds to step S1007.

When it is determined in step S1102 that the acquired data is less than the threshold value (in the case of NO in step S1102), the process proceeds to step S1106, "0" is input into the counter i, and the process proceeds to step S1107.

In step S1107, it is determined that the early warning detecting unit 134 does not detect an early warning (or some abnormality).

In step S1108, the early warning detecting unit 134 determines whether the early warning detecting process ends, and returns to step S1101 when it is determined that the early warning detecting process is to be continued (in the case of NO in step S1108). On the other hand, in Step S1108, when it is determined that the early warning detecting process is to be ended (in the case of YES in Step S1108), the early warning detecting process ends.

[Summary]

As is obvious from the above description, the abnormality detecting system according to the first embodiment performs the following process.

States of healthy cow in each time range are identified on the basis of time-series data representing a three-dimensional acceleration as measured by an acceleration sensor placed on a neck of healthy cow. State transition probability data is generated by calculating a transition probability from a state at a predetermined timing of each identified time range to a next state.

States of cow that is a monitoring target in each time range are identified based on time-series data representing a three-dimensional acceleration as measured by an acceleration sensor placed on a neck of the cow that is a monitoring target. State transition probability data is generated by calculating the transition probability from a state at a predetermined timing of the identified time range to the next state.

Based on the state transition probability data generated for healthy cow and the state transition probability data calculated for the cow that is a monitoring target, a score representing an abnormality of a state transition is calculated for each transition probability.

The maximum score is extracted from the score calculated for each transition probability, and is output as data representing the abnormality.

It is determined that the abnormality in the cow that is a monitoring target is detected, when the data representing the abnormality satisfies a prescribed condition.

Thus, in the abnormality detecting system according to the first embodiment, a score is calculated based on the state transition probability data of the cow that is a monitoring target and healthy cow to detect a change in a tendency of movement occurring prior to development of BRD (or BRDC) in contrast to the tendency of movement of healthy cow. Therefore, by the abnormality detecting system according to the first embodiment, it is possible to detect an early warning (or some abnormality) before the cow that is a monitoring target develops BRD (or BRDC).

That is, according to the first embodiment, an abnormality detecting system for detecting an abnormality of cow that is a monitoring target can be provided.

[Second Embodiment]

In the first embodiment described above, the score was calculated based on the state transition probability data of the cow that is a monitoring target and the state transition probability data of healthy cow.

However, the method for calculating the score is not limited, and, for example, the score may be calculated based on the state transition probability data of the cow that is a monitoring target. In other words, rather than detecting a change in the tendency of movement based on the contrast to the state transition probability data of healthy cow, a change in the tendency of movement may be detected based on the state transition probability data of only the cow that is a monitoring target. Hereinafter, the second embodiment will be described focusing on the differences from the first embodiment described above.

<System Configuration of Abnormality Detecting System and Functional Configuration of Server apparatus>

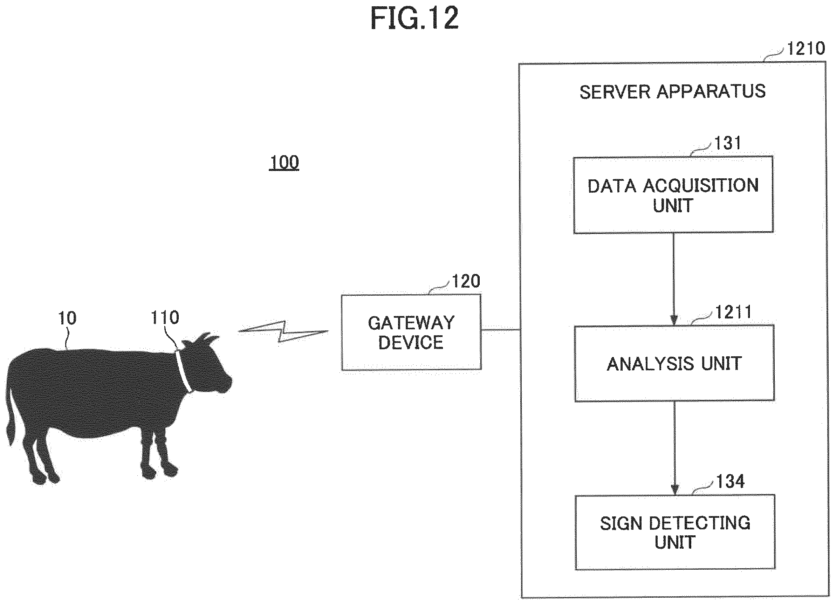

First, a system configuration of the abnormality detecting system and a functional configuration of the server apparatus will be described. Fig. 12 is a second diagram illustrating an example of the system configuration of the abnormality detecting system and the functional configuration of the server apparatus.

As shown in Fig. 12, since the system configuration of the abnormality detecting system is the same as the system configuration of the abnormality detecting system illustrated in Fig. 1, a description of the system configuration will be omitted, and the functional configuration of the server apparatus 1210 will be described here.

As illustrated in Fig. 12, the server apparatus 1210 functions as a data obtaining unit 131, an analysis unit 1211, and an early warning detecting unit 134 when an abnormality detecting program is executed. Among these units, the data obtaining unit 131 and the early warning detecting unit 134 are the same as the data obtaining unit 131 and the early warning detecting unit 134 illustrated in Fig. 1, and therefore the description thereof will be omitted here.

The analysis unit 1211 generates state transition probability data of the cow that is a monitoring target based on the time-series data of the cow that is a monitoring target among the time-series data representing the three-dimensional acceleration acquired by the data obtaining unit 131. The analysis unit 1211 calculates the score indicating

a degree of increase in each transition probability of the cow that is a monitoring target, or
a degree of decrease in each transition probability of the cow that is a monitoring target, based on the state transition probability data of the cow that is a monitoring target (i.e., the abnormality in the state transition).

<Details of Functional Configuration of Analysis unit>

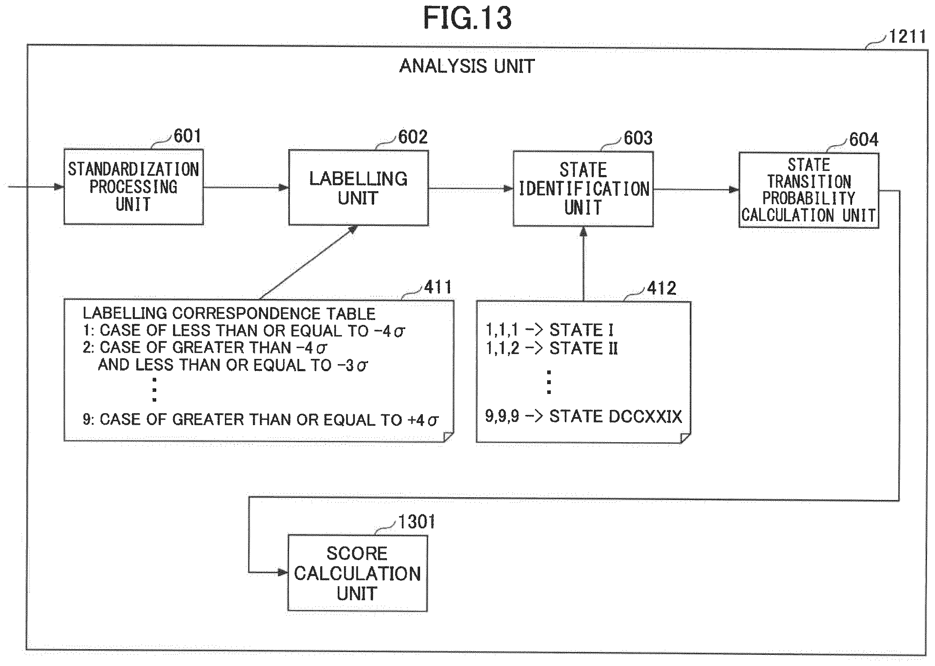

Next, the functional configuration of the analysis unit 1211 will be described in detail. Fig. 13 is a diagram illustrating a detailed functional configuration of the analysis unit. As shown in Fig. 13, the analysis unit 1211 includes a standardization processing unit 601, a labeling unit 602, a state identification unit 603, a state transition probability calculation unit 604, and a score calculation unit 1301 (a second score calculation unit).

The functions of the respective units of from the standardization processing unit 601 to the state transition probability calculation unit 604 are the same as the functions of the units of from the standardization processing unit 401 to the state transition probability calculation unit 404 of the reference data calculation unit 132 described with reference to Fig. 4. Accordingly, the score calculation unit 1301 will be described here.

The score calculation unit 1301 acquires the state transition probability data of the cow that is a monitoring target generated by the state transition probability calculation unit 604. The score calculation unit 605 calculates the score using the following equation (2) based on the state transition probability data of the cow that is a monitoring target,

(Equation 2)

$$\text{score} = -\log(X).$$

In equation (2) above, X represents the state transition probability data of the cow that is a monitoring target.

The score calculation unit 1301 calculates a score indicating the degree of increase or decrease in each transition probability included in the state transition probability data of the cow that is a monitoring target for each transition probability.

Furthermore, the score calculation unit 1301 extracts the maximum score from among the scores calculated for each of the transition probabilities, and outputs the maximum score as data indicating the abnormality of the cow that is a monitoring target at the relevant date.

<Example of process in the analysis unit>

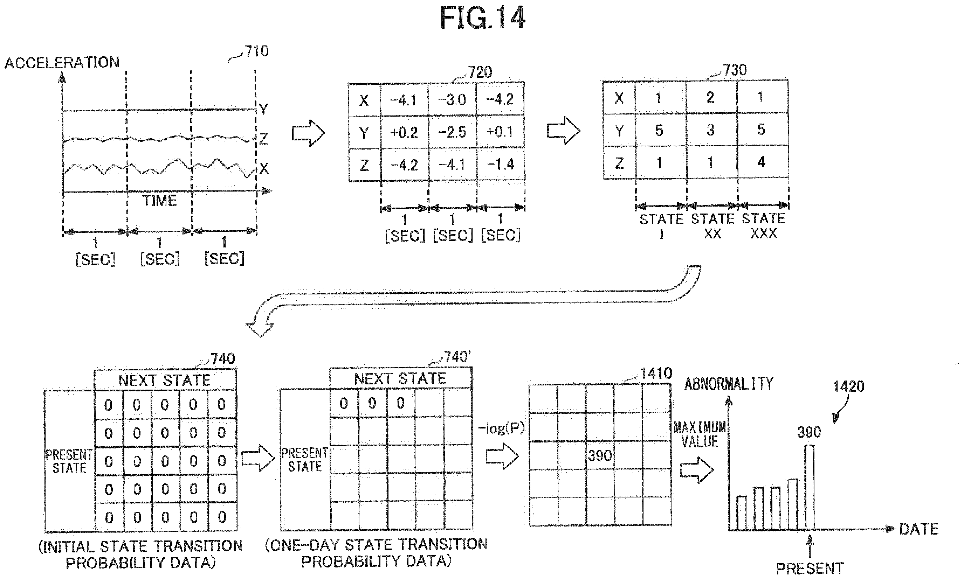

Next, a specific example of the process of the analysis unit 1211 among the process of the units executed by the server apparatus 1210 will be described. Fig. 14 is a diagram illustrating a specific example of the process of the analysis unit.

As shown in Fig. 14,

a set of time-series data 710,
standardized data 720,
labeling data 730,
initial state transition probability data 740, and
one-day state transition probability data 740',

which are acquired or generated for the cow that is a monitoring target by the standardization processing unit 601, the labeling unit 602, the state identification unit 603, and the state transition probability calculation unit 604, are the same as

the set of time-series data 510,
the standardized data 520,
the labeling data 530,

which are acquired or generated for healthy cow by the standardization processing unit 401, the labeling unit 402, the state identification unit 403, and the state transition probability calculation unit 404 obtain or generate for healthy cow, or

the initial state transition probability data 740, and
the one-day state transition probability data 740',

which are acquired or generated for the cow that is a monitoring target by the state transition probability calculation unit 604. Thus, the detailed description will be omitted here.

Also in Fig. 14, only five current states and five subsequent states are shown as the state transition probability data 740 and 740' due to the limited space in the figure.

By generating the one-day state transition probability data 740' of the cow that is a monitoring target, the score calculation unit 1301 calculates the score for each transition probability using the above equation (2) as shown in Fig. 14. Thus, the score data 1410 is generated.

As shown in Fig. 14, the score calculation unit 1301 extracts the maximum score from among the scores for each of the transition probabilities included in the score data 1410 and outputs the extracted maximum score as data indicating the abnormality of the cow that is a monitoring target at the relevant date. In Fig. 14, a graph 1420 is a graphical representation of the data representing the abnormality at each date of the cow that is a monitoring target, with the horizontal axis representing the date and the vertical axis representing the abnormality. The graph 1420 shows that the data indicating the current abnormality is "390".

<Flow of Analysis Process>

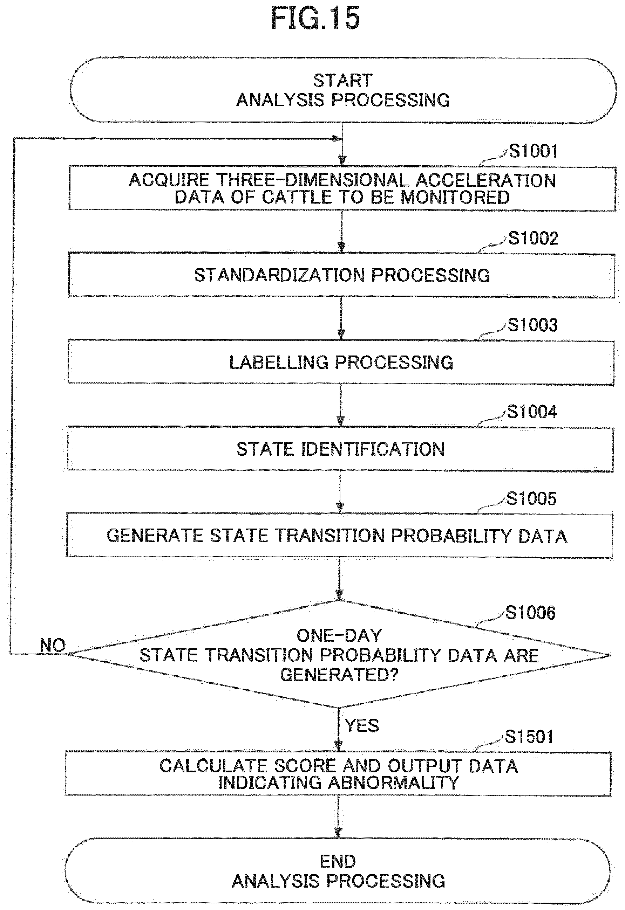

Next, the flow of the analysis process by the server apparatus 1210 will be described. Fig. 15 is a second flowchart showing the flow of the analysis process.

Among the process shown in Fig. 15, in each process of step S1001 to step S1006, the same process as in each process of step S1001 to step S1006 illustrated in Fig. 10 is performed. Therefore, the description thereof will be omitted here.

In step S1501, the score calculation unit 1301 of the analysis unit 1211 calculates the score for each transition probability based on the one-day state transition probability data of the cow that is a monitoring target. The score calculation unit 1301 of the analysis unit 1211 extracts the maximum score from among the scores calculated for each of the transition probabilities, and outputs it as data representing the abnormality of the cow that is a monitoring target at the relevant date.

[Summary]

As is obvious from the above description, the abnormality detecting system according to the second embodiment performs the following process.

States in each time range of cow that is a monitoring target is identified based on time-series data representing a three-dimensional acceleration as measured by an acceleration sensor placed on a neck of the cow that is a monitoring target. State transition probability data is generated by calculating a transition probability from a state at a predetermined timing of an identified time range to a next state.

Based on the state transition probability data generated for the cow that is a monitoring target, a score representing an abnormality of a state transition is calculated for each stage transition probability.

The maximum score is extracted from the score calculated for each transition probability, and is output as data indicating the abnormality.

It is determined that the abnormality in the cow that is a monitoring target is detected, when the data representing the abnormality satisfies a prescribed condition.

Thus, in the abnormality detecting system in accordance with the second embodiment, a score is calculated based on the state transition probability data of the cow that is a monitoring target to detect a change in a tendency of movement occurring prior to development of BRD (or BRDC). Therefore, by the abnormality detecting system according to the second embodiment, it is possible to detect an early warning (or some abnormality) of BRD (or BRDC) before the cow that is a monitoring target develops BRD (or BRDC) as in the first embodiment.

That is, according to the second embodiment, an abnormality detecting system for detecting an abnormality of cow that is a monitoring target can be provided.

[Third Embodiment]

In the first and second embodiments described above, an acceleration sensor is placed on the neck portion, but the attachment part of the acceleration sensor is not limited to the neck portion. The acceleration sensor may be placed on other parts.

Although in the above-described first and second embodiments described above an acceleration sensor is attached as a motion sensor, a motion sensor other than the acceleration sensor (e.g., an angular velocity sensor) may be attached.

In the first embodiment described above, the state transition probability data for healthy cow was described as generating state transition probability data for a certain period of time. However, the period may be one day or a plurality of days.

The state transition probability data for healthy cow may be obtained by generating state transition probability data for one healthy cow, or by generating state transition probability data for a plurality of cows and calculating an average of the data.

In the above-described first and second embodiments, it was described that data indicating abnormalities was output once every day for the cow that is a monitoring target, and it was determined whether an early warning (or some abnormality) was detected once every day. However, the output frequency for outputting data indicating abnormality and the decision frequency for determining whether an early warning (or some abnormality) is detected are not limited to one day, but may be less than one day or greater than or equal to one day.

In the above-described first and second embodiments, the case in which time-series data representing a three-dimensional acceleration was used was described. However, the number of axial directions using time-series data is not limited to three axes. For example, time-series data representing an acceleration of any two axes may be used. Alternatively, time-series data representing only a single-axis acceleration may be used.

In the above-described first and second embodiments, it was described that nine types of labels "1" to "9" were used for the labeling process. However, the types of labels used for the labeling process are not limited to nine types.

In the above-described first and second embodiments, the time range at the time of the standardization process is set to "1 [SEC]", but the time range at the time of the standardization process is not limited to "1 [SEC]".

In the above-described first and second embodiments, it was described that when the data indicating the abnormality was greater than or equal to the threshold value for two consecutive days, it was determined that an early warning (or some abnormality) was detected. However, the conditions for determining whether an early warning (or some abnormality) is detected are not limited thereto, and other predetermined conditions may be used to perform the determination. In other words, it may be determined based on whether pre-defined conditions are satisfied or not.

In the above-described first and second embodiments, it was described that the animals that is a monitoring target were cow, but animals other than cow may be monitored.

It should be noted that the present invention is not limited to the above-described embodiments. An embodiment where another element is used in a combination, for example, may be covered by the present invention. In these respects, it is possible to change the embodiment in any way without departing from the spirit of the invention, and how to change can be appropriately determined in accordance with what the present invention is applied to.

The present application claims priority to Japanese Patent Application No. 2020-040117, filed March 9, 2020, and the entire contents of Japanese Patent Application No. 2020-040117 are incorporated herein by reference in its entirety.

[Description of Symbols]

- 100: Abnormality detecting system
- 110: Measuring device
- 130: Server apparatus
- 131: Data obtaining unit
- 132: Reference data calculation unit
- 133: Analysis unit
- 134: Early warning detecting unit
- 150,160: Terminal device
- 401: Standardization processing unit
- 402: Labeling unit
- 403: State identification unit
- 404: State transition probability calculation unit
- 601: Standardization processing unit
- 602: Labeling unit
- 603: State identification unit
- 604: State transition probability calculation unit
- 605: Score calculation unit
- 1211: Analysis unit

1301: Score calculation unit

## Claims

1. An abnormality detecting system comprising:

   a first identification unit configured to identify a state of an animal that is a monitoring target in each time range, based on time-series data from a motion sensor placed on a predetermined portion of the animal that is a monitoring target;
   a first calculation unit configured to calculate a transition probability from the state at a predetermined timing of each time range identified by the first identification unit to a next state; and
   a determining unit configured to determine that an abnormality of the animal that is a monitoring target is detected when a score calculated based on the transition probability to the next state satisfies a predetermined condition.

2. The abnormality detecting system as claimed in claim 1 further comprising:

   a first standardization processing unit configured to perform a standardization process for the time-series data from the motion sensor placed on the predetermined portion of the animal that is a monitoring target in each time range; and
   a first labeling unit configured to perform a labeling process for a result of the standardization process performed in each time range by the first standardization processing unit, wherein
   the first identification unit is configured to identify the state in each time range from a combination of results of the labeling process performed by the first labeling unit.

3. The abnormality detecting system as claimed in claim 2 further comprising:

   a first score calculation unit configured to calculate the score based on each transition probability calculated by the first calculation unit, and extract a maximum score from among the calculated scores, wherein
   the determining unit is configured to determine that an abnormality of the animal that is a monitoring target is detected when the score extracted by the first score calculation unit is greater than or equal to a predetermined threshold value for consecutive days.

4. The abnormality detecting system as claimed in claim 1 further comprising:

   a second identification unit configured to identify a state of a healthy animal in each time range, based on time-series data from a motion sensor placed on a predetermined portion of the healthy animal;
   a second calculation unit configured to calculate a transition probability from the state at a predetermined timing of each time range identified by the second identification unit to a next state; and
   a second score calculation unit configured to calculate the score based on each transition probability calculated by the first calculation unit and each transition probability calculated by the second calculation unit, and extract a maximum score from among the calculated scores.

5. The abnormality detecting system as claimed in claim 4, wherein
   the determining unit is configured to determine that an abnormality of the animal that is a monitoring target is detected when the score extracted by the second score calculation unit is greater than or equal to a predetermined threshold value for consecutive days.

6. The abnormality detecting system as claimed in claim 4 further comprising:

   a second standardization processing unit configured to perform a standardization process for the time-series data from the motion sensor placed on the predetermined portion of the healthy animal in each time range; and
   a second labeling unit configured to perform a labeling process for a result of the standardization process performed in each time range by the second standardization processing unit, wherein
   the second identification unit is configured to identify the state in each time range from a combination of results of the labeling process performed by the second labeling unit.

FIG.1
100
10
110
GATEWAY DEVICE
120
SERVER APPARATUS
130
DATA ACQUISITION UNIT
131
REFERENCE DATA CALCULATION UNIT
132
ANALYSIS UNIT
133
SIGN DETECTING UNIT
134
ACCELERATION DATA STORAGE UNIT
135
REFERENCE DATA STORAGE UNIT
136

FIG.2
130
SERVER APPARATUS
201
PROCESSOR
202
MEMORY
203
AUXILIARY STORAGE DEVICE
204
I/F DEVICE
205
COMMUNICATION DEVICE
206
DRIVE DEVICE
207
210
OPERATION DEVICE
211
DISPLAY DEVICE
212
RECORDING MEDIUM

FIG.3

ACCELERATION
Y
Z
X
TIME
131
DATA ACQUISITION UNIT
135
ACCELERATION DATA STORAGE UNIT
HEALTHY CATTLE

FIG.4
132
REFERENCE DATA CALCULATION UNIT
135
ACCELERATION DATA STORAGE UNIT
136
REFERENCE DATA STORAGE UNIT
401
STANDARDIZATION PROCESSING UNIT
402
LABELLING UNIT
403
STATE IDENTIFICATION UNIT
404
STATE TRANSITION PROBABILITY CALCULATION UNIT
411
LABELLING CORRESPONDENCE TABLE
1: CASE OF LESS THAN OR EQUAL TO −4σ
2: CASE OF GREATER THAN −4σ AND LESS THAN OR EQUAL TO −3σ
9: CASE OF GREATER THAN OR EQUAL TO +4σ
412
1,1,1 -> STATE I
1,1,2 -> STATE II
9,9,9 -> STATE DCCXXIX

ACCELERATION
510
Y
Z
X
TIME
1 [SEC]
1 [SEC]
1 [SEC]
520
X -4.1 -4.0 -4.2
Y +0.8 -1.1 -1.2
Z -4.2 -4.1 -3.4
1 [SEC]
1 [SEC]
1 [SEC]
530
X 1 1 1
Y 5 4 4
Z 1 1 2
STATE I
STATE V
STATE IIV
541
542
540
NEXT STATE
STATE I
STATE II
STATE III
STATE DCCXXIX
TOTAL
PRESENT STATE
STATE I
STATE II
STATE III
STATE DCCXXIX
1
1
1
1
TRANSITION PROBABILITY = EACH TRANSITION FREQUENCY / TOTAL NUMBER OF TRANSITION FREQUENCY
STATE TRANSITION PROBABILITY DATA OF HEALTHY CATTLE

FIG.5

FIG.6

ANALYSIS UNIT
133
601
STANDARDIZATION PROCESSING UNIT
602
LABELLING UNIT
603
STATE IDENTIFICATION UNIT
604
STATE TRANSITION PROBABILITY CALCULATION UNIT
411
LABELLING CORRESPONDENCE TABLE
1: CASE OF LESS THAN OR EQUAL TO −4σ
2: CASE OF GREATER THAN −4σ AND LESS THAN OR EQUAL TO −3σ
⋮
9: CASE OF GREATER THAN OR EQUAL TO +4σ
412
1,1,1 -> STATE I
1,1,2 -> STATE II
⋮
9,9,9 -> STATE DCCXXIX
SCORE CALCULATION UNIT
605
136
REFERENCE DATA STORAGE UNIT

ACCELERATION
710
Y
Z
X
TIME
1 [SEC]
1 [SEC]
1 [SEC]
720
X -4.1 -3.0 -4.2
Y +0.2 -2.5 +0.1
Z -4.2 -4.1 -1.4
1 [SEC] 1 [SEC] 1 [SEC]
730
X 1 2 1
Y 5 3 5
Z 1 1 4
STATE I
STATE XX
STATE XXX
740
NEXT STATE
PRESENT STATE
(INITIAL STATE TRANSITION PROBABILITY DATA)
740'
NEXT STATE
PRESENT STATE
(ONE-DAY STATE TRANSITION PROBABILITY DATA)
-log(P∘X)
750
410
MAXIMUM VALUE
ABNORMALITY
760
410
DATE
PRESENT
540
NEXT STATE
PRESENT STATE
STATE TRANSITION PROBABILITY DATA OF HEALTHY CATTLE

FIG.7

800
480
420
410
ABNORMALITY
THRESHOLD VALUE
DATE
803
802
801
DETECTING

FIG.8

FIG.9
START
REFERENCE DATA CALCULATION PROCESSING
S901
ACQUIRE THREE-DIMENSIONAL
ACCELERATION DATA OF HEALTHY CATTLE
S902
STANDARDIZATION PROCESSING
S903
LABELLING PROCESSING
S904
STATE IDENTIFICATION
S905
GENERATE STATE TRANSITION PROBABILITY DATA
S906
STORE AS REFERENCE DATA
S907
COMPLETED?
NO
YES
END
REFERENCE DATA CALCULATION PROCESSING

# FIG.10

START
ANALYSIS PROCESSING
S1001
ACQUIRE THREE-DIMENSIONAL ACCELERATION DATA OF CATTLE TO BE MONITORED
S1002
STANDARDIZATION PROCESSING
S1003
LABELLING PROCESSING
S1004
STATE IDENTIFICATION
S1005
GENERATE STATE TRANSITION PROBABILITY DATA
S1006
ONE-DAY STATE TRANSITION PROBABILITY DATA ARE GENERATED?
NO
YES
S1007
ACQUIRE STATE TRANSITION PROBABILITY DATA OF CATTLE TO BE MONITORED AND REFERENCE DATA
S1008
CALCULATE SCORE AND OUTPUT DATA INDICATING ABNORMALITY
END
ANALYSIS PROCESSING

# FIG.11

START
SIGN DETECTING
PROCESSING
ACQUIRE DATA INDICATING
ABNORMALITY
S1101
S1102
YES
THRESHOLD
VALUE OR MORE?
NO
S1103
i=i+1
S1106
i=0
S1104
i IS
PREDETERMINED NUMBER
OR MORE?
NO
YES
S1105
DETERMINE THAT SIGN
IS PRESENT
S1107
DETERMINE THAT SIGN
IS ABSENT
S1108
NO
END?
YES
END
SIGN DETECTING
PROCESSING

100
10
110
120
GATEWAY DEVICE
1210
SERVER APPARATUS
131
DATA ACQUISITION UNIT
1211
ANALYSIS UNIT
134
SIGN DETECTING UNIT

FIG.12

FIG.13
1211
ANALYSIS UNIT
601
STANDARDIZATION PROCESSING UNIT
602
LABELLING UNIT
603
STATE IDENTIFICATION UNIT
604
STATE TRANSITION PROBABILITY CALCULATION UNIT
411
LABELLING CORRESPONDENCE TABLE
1: CASE OF LESS THAN OR EQUAL TO −4σ
2: CASE OF GREATER THAN −4σ AND LESS THAN OR EQUAL TO −3σ
9: CASE OF GREATER THAN OR EQUAL TO +4σ
412
1,1,1 -> STATE I
1,1,2 -> STATE II
9,9,9 -> STATE DCCXXIX
1301
SCORE CALCULATION UNIT

FIG.14
710
ACCELERATION
TIME
Y
Z
X
1 [SEC]
1 [SEC]
1 [SEC]
720
X -4.1 -3.0 -4.2
Y +0.2 -2.5 +0.1
Z -4.2 -4.1 -1.4
1 [SEC] 1 [SEC] 1 [SEC]
730
X 1 2 1
Y 5 3 5
Z 1 1 4
STATE I
STATE XX
STATE XXX
740
NEXT STATE
PRESENT STATE
(INITIAL STATE TRANSITION PROBABILITY DATA)
740'
NEXT STATE
PRESENT STATE
(ONE-DAY STATE TRANSITION PROBABILITY DATA)
-log(P)
1410
390
MAXIMUM VALUE
1420
ABNORMALITY
390
DATE
PRESENT

FIG.15
START
ANALYSIS PROCESSING
S1001
ACQUIRE THREE-DIMENSIONAL ACCELERATION DATA OF CATTLE TO BE MONITORED
S1002
STANDARDIZATION PROCESSING
S1003
LABELLING PROCESSING
S1004
STATE IDENTIFICATION
S1005
GENERATE STATE TRANSITION PROBABILITY DATA
S1006
ONE-DAY STATE TRANSITION PROBABILITY DATA ARE GENERATED?
NO
YES
S1501
CALCULATE SCORE AND OUTPUT DATA INDICATING ABNORMALITY
END
ANALYSIS PROCESSING

## INTERNATIONAL SEARCH REPORT

International application No. PCT/JP2021/008052

A. CLASSIFICATION OF SUBJECT MATTER
A01K 29/00(2006.01)i
FI: A01K29/00 A

According to International Patent Classification (IPC) or to both national classification and IPC

B. FIELDS SEARCHED

Minimum documentation searched (classification system followed by classification symbols)
A01K29/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2019-122368 A (DESAMIS CO., LTD.) 25 July 2019 (2019-07-25) entire text, all drawings | 1-6 |
| A | WO 2018/012005 A1 (SEIKE, Kouji) 18 January 2018 (2018-01-18) entire text, all drawings | 1-6 |
| A | JP 5156030 B2 (SAY SYSTEMS LIMITED) 06 March 2013 (2013-03-06) entire text, all drawings | 1-6 |
| A | WO 2011/120529 A1 (KOEBENHAVNS UNIVERSITET) 06 October 2011 (2011-10-06) entire text, all drawings | 1-6 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 May 2021 (12.05.2021) | 25 May 2021 (25.05.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/008052

| Patent Documents referred in the Report | Publication Date | Patent Family | Publication Date |
|---|---|---|---|
| JP 2019-122368 A | 25 Jul. 2019 | (Family: none) | |
| WO 2018/012005 A1 | 18 Jan. 2018 | (Family: none) | |
| JP 5156030 B2 | 06 Mar. 2013 | US 2010/0321189 A1 entire text, all drawings WO 2008/097111 A1 | |
| WO 2011/120529 A1 | 06 Oct. 2011 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2020040117 A